# EUROPEAN PATENT SPECIFICATION

(11) **EP 2 717 863 B1**
(45) Date of publication and mention of the grant of the patent: **27.02.2019**
(21) Application number: 12725023.1
(22) Date of filing: 01.06.2012
(51) Int. Cl.: A61K 9/50

(54) **CORE-SHELL CAPSULES**
KERNHÜLLENKAPSELN
CAPSULES COEUR/ENVELOPPE

(30) Priority: 07.06.2011 EP 11168960
(43) Date of publication of application: 16.04.2014
(73) Proprietor: Firmenich SA, 1211 Geneva 8 (CH)
(72) Inventor: DARDELLE, Gregory, CH-1211 Geneva 8 (CH); ERNI, Philipp, CH-1211 Geneva 8 (CH)
(74) Representative: Rupp, Christian
(86) International application number: PCT/EP2012/060339
(87) International publication number: WO 2012/168144

(56) References cited:
- WO-A1-2004/103351
- US-A1- 2002 172 716
- US-A1- 2010 143 422
- DAI ZHIFEI ET AL: "Novel capsules with high stability and controlled permeability by hierarchic templating", ANGEWANDTE CHEMIE, WILEY - V C H VERLAG GMBH & CO. KGAA, DE, vol. 41, no. 21, 1 January 2002 (2002-01-01), pages 4019-4022, XP002299226, ISSN: 0044-8249, DOI: 10.1002/1521-3773(20021104)41:21<4019::AID -ANIE4019>3.0.CO;2-Z

## Description

### Technical Field

The present invention relates to a core-shell delivery system, a method for preparing a delivery system and the use of silica in the shell of a delivery system.

### Background and Prior Art

Hydrogel capsules are water-swollen cross-linked polymeric structures that are typically used when there is a need to provide a stable environment for a volatile or oxygen-sensitive active ingredient in contact with a high moisture environment.

They are used in medical applications and, more recently, in flavor and fragrance applications. For instance WO-A1-2006/119660 and EP-A1-1841521 (both to Givaudan) disclose hydrogel shells encapsulating volatile substances such as flavors and fragrances.

Hydrogel capsules are typically formed by the process of coacervation, about which many publications exist, such as WO-A1-96/32017 (Tastemaker), FR1165805 (The National Cash Register Company), MX9704934 (Tastemaker Corporation) and US6325951 (Givaudan).

In the standard complex coacervation process, there is separation of a colloid into a colloid-rich phase (the coacervate) and an aqueous solution of the coacervating agent (the equilibrium liquid), forming an oil coated with protein, carbohydrate, or polymeric droplets so as to suspend the oil in water. The phases are absorbed into one lipid phase and one aqueous phase. The first lipid phase forms the core which is surrounded by a hydrogel capsule. The hydrogel capsule is a colloid in which the dispersed phase has combined with the continuous phase (water) to produce a viscous gelatinous product.

Whilst coacervation is useful for encapsulating active ingredients such as flavors and fragrances, the gel-like nature of conventional coacervates limits their applicability. It would therefore be desirable to address this problem.

In particular, classical coacervate capsules remain resistant to mechanical rupture under both small and large mechanical deformations. However, while mechanical resistance at small loads (such as those encountered during storage or processing) is desirable, mechanical resistance becomes problematic if it prevents the intended rupture of the capsule under strong deformations such as those encountered during chewing, rubbing, or squeezing the capsule in the final application (where the capsule is expected to burst, rather than to be mechanically resistant). Under those "high deformation" conditions, classical coacervate capsules are deformed in a manner similar to a crosslinked "rubber", but it would be advantageous to have capsules that behave like a "rigid" and brittle solid shell at high loads.

Capsules with desirable mechanical properties in the context of this invention are therefore capsules that are resistant to weak mechanical loads, but that can be made to burst easily when subjected to an imposed deformation.

The use of Si(OEt)₄ (hereinafter also referred to as "TEOS") in the formation of capsules has been described in the publication "Synthesis of Mesoporous Silica Nanospheres Promoted by Basic Amino Acids and their Catalytic Application", Toshiyuki Yokoi, Takumi Karouji, Seigo Ohta, Junko N. Kondo and Takashi Tatsumi, Chem. Mater., DOI: 10.1021/cm9037846. Here, it is described that discrete mesoporous silica nanospheres with a narrow size distribution can be prepared by a method based on an emulsion system containing Si(OEt)₄, water, a cationic surfactant, and a basic amino acid under weakly basic conditions (p.H. 9 to 10). This is clearly unrelated to hydrogel capsules.

US 7611731 (SBA Materials Inc., USA) discloses mesoporous polymer/inorganic oxide hybrid materials based on silica/amphiphilic block copolymer monoliths that have an ordered silica/polymer architecture.

GB 2473870 (Givaudan) describes a dispersion of active-containing microbeads within a non-aqueous continuous phase, the microbeads comprising a plurality of droplets within a hybrid matrix. In this publication the matrix comprises both a hydrophilic organic material and an inorganic oxide that forms a continuous network through the organic material. This is unrelated to core/shell capsules containing a single core *comprising a solid active ingredient and*/*or a liquid active ingredient, and* a composite shell. Furthermore, this publication describes dispersions of microbeads in a non-aqueous continuous phase but it does not describe capsules that are easily obtained in a dry form, or in an aqueous slurry or suspension.

US2010/0143422A1 describes microcapsules composed of a sol-gel material that contain active ingredients. The examples given therein are based on emulsification of fragrance oils containing TEOS, emulsification of fragrance oils with subsequent addition of TEOS, or emulsification of fragrance oils with subsequent addition of TEOS where the TEOS has been previously hydrolyzed. The structure of the microcapsules consists of a fragrance core and a sol-gel wall material. The process, which consists of precipitation, is not confined to the scaffold of a hydrogel shell and so does not allow for localized and controlled formation of the TEOS inorganic phase. It also may result in excessive precipitation in a core liquid. It would be desirable to avoid such drawbacks.

Moreover, said document discloses a shell which is made only of silica and although silica-only microcapsules, which are brittle, might offer good mechanical properties for the release of the active ingredient, it is known to the experimenter skilled in the art that microcapsules with silica-only shells are highly porous material, and as such it is a poor protective barrier for active ingredients such as volatile perfume oils or flavors. The prior art document suggests to solve this problem by adding a spray-drying step to entrap the core-shell capsules into an additional starch matrix.

However, while matrix encapsulates are known to provide good barrier properties, they do not possess any of the highly desirable mechanical properties of core/shell capsules for a controlled release. In particular, it would be desirable to have a capsule that provides both excellent barrier properties during storage and processing of the final product (such a food or consumer product) while also providing the mechanical properties needed for a "burst"-type release upon fracture.

Our present invention overcomes the above-mentioned drawbacks by providing a core/shell capsule with a composite shell comprising an organic and an inorganic phase based on polymeric strands with silica interspersed therein.

WO-A1-2009/147119 (Symrise GmbH & CO KG) describes a capsule, comprising or consisting of a core and a shell surrounding the core, wherein the shell comprises a polymeric material or consists thereof, which polymeric material can be produced by reacting a component (A) with a component (B), wherein component (A) consists of polysiloxanes bearing one or more amino groups and component (B) consists of one or more polyisocyanates. Thus, silicon is part of the chemical composition of the polymer.

In the publication "Facile method for preparing organic/inorganic hybrid capsules using amino-functional silane coupling agent in aqueous media" by Kurayama et al, Journal of Colloid and Interface Science 2010, 349(1), pages 70 to 76, there is described organic-inorganic hybrid capsules formed using amino-functional silane coupling agent (3-aminopropyltriethoxysilane, "APTES") together with alginate capsules in an aqueous media toi forma hybrid shell ("AP-capsule"). The authors propose that it is the interaction of the carboxylate groups of an alginate and the protonated amino groups of APTES that enable the formation of the AP-capsule.

WO 2004/103351 A1 (Tirelli and Cellesi, The University of Manchester, UK) describes carrier particles with a cavity containing an active ingredient, surrounded by a polymer-based hydrogel membrane. Also described is an intermediate structure needed to make such capsules, for which a central silica particle is first formed, in which the active ingredient is contained. This silica particle forms the core of the intermediate structure, and it is coated with a polymer shell (significantly different from the ones of the present invention), which is then crosslinked. At this stage, the intermediate product is a silica particle containing an active ingredient, surrounded by an organic polymer layer which is not interspersed with the silica layer. To obtain the final structure, the silica in the sacrificial core is dissolved, leaving behind a polymer-based shell surrounding the cavity with the active.

Neither the intermediate product nor the final product contains silica in the shell of the capsule. Silica is only part of the sacrificial core template and is later dissolved. The barrier and mechanical properties of the capsule are therefore those of the organic polymer shell alone. Since such hydrogel shells alone are known to be permeable and poorly suited for volatile ingredients such as flavor and fragrance molecules, it would be desirable to overcome this physical limitation and make core/shell capsules wherein the shell becomes a dense, reinforced barrier superior to a traditional hydrogel shell.

It becomes obvious from the above review of the background and prior art that classical hydrogel and coacervate capsules do not possess the mechanical properties desired for a capsule that provides a good protective barrier and is mechanically resistant to small loads, but that easily bursts as it is deformed.

The present invention seeks to provide a new hydrogel encapsulation system that addresses one or more of the problems highlighted above. Preferably, the present invention addresses the problems associated with conventional hydrogel systems, such as lack of ridigity.

### Summary of the Invention

Accordingly, the invention provides a process for preparing a core-shell capsule comprising the steps of:
(i) mixing a solid active ingredient and/or an oily liquid active ingredient with a polymeric material capable of forming a hydrogel shell around the active ingredient(s);
(ii) forming a shell comprising a hydrogel scaffold formed of a polymeric lattice around the core;
(iii) optionally cross-linking the polymeric lattice;
(iv) contacting the optionally cross-linked core-shell hydrogel shell with a liquid silica precursor so as to cause precipitation of silica particles within the scaffold structure thereby forming a composite shell of silica particles interspersed between the polymeric lattice.

The present invention further provides a core-shell capsule wherein
(a) the shell is a composite comprising an organic and an inorganic phase, the organic phase comprising a network of polymeric strands and the inorganic phase comprising silica, and wherein the inorganic phase is physically interspersed between at least a proportion of the organic phase; and
(b) the core comprises a solid active ingredient and/or an oily liquid active ingredient.

Preferably, the composite shell provides a barrier against loss of the active ingredient into the environment.

Preferably, the composite shell provides excellent mechanical stability to the capsule under small mechanical loads but easily bursts to allow release of the ingredient under higher mechanical loads.

"Oily" in the context of this invention means that the liquid active ingredient in water is fully or partially immiscible with water so that it can be dispersed in the form of discrete emulsion drops within an aqueous phase during the manufacturing of the capsules.

Preferably, oily means that the liquid active ingredient has an interfacial tension against water of at least 0.0001 N/m, preferably an interfacial tension of at least 0.001 N/m.

Preferably, the oily liquid typically contains at least one component for which the value of the base-ten logarithm of the partition coefficient between octanol and water (logP) is greater than 4; the oil may contain additional components with logP less than 4.

In another aspect, the invention provides the use of silica physically interspersed in the shell portion of a hydrogel core-shell capsule comprising a liquid flavor or fragrance in the core portion to increase the rigidity and/or mechanical stability of the capsule.

### Brief Description of the Drawings

Figure 1 shows the absorption of TEOS into the scaffold of a hydrogel shell;
Figure 2 shows the content of silica within a hydrogel shell;
Figure 3 shows the measured silicon content in a hydrogel shell; and
Figure 4 shows the shape of a silica/hydrogel capsule according to the invention.
Figure 5 shows the mechanical behavior of a core-shell capsule according to the invention, a traditional core-shell capsule without silica, and a matrix (non-core/shell) encapsulate. Force (normalized with the force at rupture) is plotted against deformation.

### Detailed Description of the Invention

In a first aspect the present invention relates to a method of preparing a core-shell capsule based on a hydrogel scaffold and an inorganic component that precipitates within the hydrogel scaffold to provide a capsule having excellent rigidity or mechanical stability.

The structure of the capsule may be described as a shell that comprises a composite of cross-linked polymeric units and an inorganic solid material interspersed between at least some of the polymeric units.

The inorganic solid material is preferably not chemically bound to the polymeric units but is physically interspersed therein. This can be achieved by forming the network of polymeric units and then depositing the inorganic solid material therein.

Deposition is preferably achieved by precipitation of the inorganic solid material or particles on or in the pre-formed gelatinous network of polymeric strands.

Thus, the process of the present invention preferably comprises the steps of:
(i) forming a hydrogel scaffold comprising a polymeric lattice;
(ii) optionally cross-linking the polymeric lattice;
(iii) contacting the cross-linked hydrogel capsule with a liquid silica precursor so as to cause precipitation of silica particles within the scaffold structure.

The following paragraphs describe features of the process as well as features of the capsule. For the avoidance of doubt, such features may be used interchangeably. Thus, features described with reference to the process may be used to further define features of the capsule and features described with reference to the capsule may be used to further define features of the process.

Silica is an essential component of the composite shell.

In order to deposit the silica so as to form the composite shell structure, the silica must be provided in a form suitable for deposition. It has been found that silica is most effectively deposited when it is generated from a liquid precursor. The liquid silica precursor is any precursor that is capable of precipitating silica upon contact with the shell scaffold. In preferred aspect, the liquid silica precursor is capable of precipitating silica upon contact with a hydrogel. More preferably it is selected from the group consisting of silicon alkoxides Si(OR)₄, partially or fully hydrolyzed forms thereof, or mixtures thereof. Examples of suitable silica precursors include dimethyldiethoxysilane DMDES ((CH3)2Si(OC2H5)2), sodium silicates, Na2SiO3, or their hydrates, Na2SiO3·nH2O, and TEOS, Si(OC2H5)4 (CAS no. 78-10-4).

Most preferably the silica precursor is TEOS. TEOS is a colorless liquid with relative molecular weight 208.33 with a relative density of 0.94, a melting point of -77 °C and a boiling point of 166-169 °C. It is readily available commercially (e.g. Sigma-Aldrich, Wacker Chemie).

The liquid precursor may be present as such, that is in its pure form. Alternatively, it can in the form of a solution. In the latter case, the solvent can be an aqueous solvent, such as water, an organic solvent, such as ethanol or propylene glycol or even a mixture of an aqueous and an organic solvent.

An example of a liquid precursor present in the form of an aqueous solution is aqueous sodium silicate. An example of a liquid precursor in the form of an ethanolic solution is a 1:1 v/v of TEOS and ethanol.

The silica is believed to be evenly distributed within the shell structure of the hydrogel scaffold and provides a structure having rigidity or mechanical stability across the surface thereof.

The polymeric component of the hydrogel shell of the present invention is typically a protein, which may be crosslinked or non-crosslinked, and/or a polymer (said polymer may also include carbohydrate moieties or be a carbohydrate).

Typical polymers useful in formation of the hydrogel shell include, in particular, negatively charged polymers. For example, they may be selected from gum arabic, xanthan, alginate salts, cellulose derivatives, for example carboxymethyl cellulose, pectinate salts, carrageenan, polyacrylic and methacrylic acid, and/or mixtures thereof. Further suitable non-proteins can be derived from the literature, for example from WO 2004/022221, page 4, lines 27-29.

Proteins, which are amino acids polymers, useful for forming the hydrogel capsule shell include albumins, vegetable globulins and gelatines. The molecular weight of the protein is typically in the order of 40'000 to 500'000 preferably 20'000 to 250'000. Some protein aggregates, however, may have molecular weights in the millions.

Gelatin is particularly preferred. Examples include fish, pork, beef, and/or poultry gelatin. According to a preferred embodiment, the protein is fish, beef or poultry gelatine.

According to an embodiment of the invention, said polymeric component comprises typically a protein (such as a gelatine) and a polymer (such as arabic gum).

In a preferred aspect, a surfactant is used in the process. Preferably the surfactant is soluble in the liquid precursor. For aqueous liquid precursors, the surfactant is ideally a water-soluble surfactant, from the classes of anionic, non-ionic, cationic, or zwitterionic surfactants. Example include, but are not limited to, sodium dodecyl sulphate, sodium laureth sulphate, polysorbates (e.g. Tween® 20); and polymeric emulsifiers such as gum arabic, milk proteins, soy proteins, modified starch.

If the precursor is TEOS or is present in an organic solvent, preferred surfactants are sorbitan esters, PGPR, mono/diglyceride esters, and phospholipids. In a most preferred aspect the precursor is TEOS and the surfactant added is lecithin, preferably soy lecithin. In such a case, the lecithin is preferably added in the form of a solution of 0.5%w/w to 5%w/w of lecithin in pure TEOS.

The surfactant may be added to the liquid precursor in order to reduce the likelihood that the capsules agglomerate during the precipitation step.

Following the precipitation step, it is preferred that the capsules are washed. The washing medium can be any suitable liquid that does not react with the capsules. Water is preferred, whilst a mixture of water and an alcohol is even more preferred. Most preferably the capsules are washed with water, an alcohol and lactic acid in order to ensure that residual TEOS precipitates onto the surface of the capsules. The advantage of the washing step is that free-flowing particles are obtained that are stable upon storage.

It is preferred that the pH of the capsule slurry is below pH 5 during washing.

Optionally the composite shell may contain residual solvent (typically water) if the process is stopped before all water in the hydrogel layer has been consumed by the precipitation.

Prior to precipitation, the polymer content of the hydrogel layer may desirably be in a range of from 0.1 up to 90% w/w, where %w/w is the mass of polymer in the hydrogel layer per mass of hydrogel, the latter consisting of polymer and solvent (typically water). Preferably, the polymer content is between 0.1 and 30%w/w and most preferably between 0.5 and 20%w/w.

After precipitation, the content of polymer in the composite organic/inorganic shell may range from 0.1%w/w to 80%w/w, where %w/w is the mass of polymer in the composite layer per mass of composite layer material, the latter consisting of polymer and inorganic precipitate. Preferably it is between 10 and 80%w/w and most preferably between 1 and 60%w/w.

The process is preferably carried out in a stirred vessel to ensure a homogeneous precipitation and good mixing, and to avoid fusion of shells of individual capsules upon contact. Additionally, the stirring process should be sufficiently mild to avoid breakage of the composite capsule shells.

The core comprises an active ingredient. It may be liquid or solid. If liquid, it is preferred that the core has a viscosity of no more than ten times the viscosity of the continuous phase. Preferably, the viscosity of the core liquid during the process is below 1 Pas and most preferably below 5000 mPas.

The active forming the core is preferably a lipophilic (oily) liquid in order to allow formation of a preliminary dispersion oil drops that are subsequently encapsulated. The active may furthermore be a solid if the solid can be dispersed in the water phase during the process, allowing it to deposit in the first step the hydrogel layer composed of water and polymer, and in the second step to form the precipitated inorganic phase within the hydrogel scaffold.

According to a particular embodiment of the invention, said active is a flavor or a perfuming composition.

The average capsule size can be between 10 micrometers and 100 micrometers, in which case the advantage is that the capsules are resistant against deformation and breakage. Most preferably, though, the capsule size is between 100 micrometers and 2 millimetres; in this latter case the advantage is that the capsules are easily breakable; additionally, the larger capsules are visually attractive and may be used to add a distinctive appearance to a product, such as, for example, body wash or liquid soap compositions.

The thickness of the composite shell is preferably between 1 and 100 micrometers, and most preferably between 10 and 50 micrometers. This range of shell thicknesses allows for capsules that are mechanically stable during processing and storage, yet easily breakable in application. Capsules with thicknesses in this range have furthermore been observed to provide excellent stability against diffusion of the active ingredient over periods up to at least 18 months when stored at room temperature, without any volumetric shrinkage of the capsule being observed.

The ratio of the capsule size to the thickness of the shell is preferably between 20:1 and 5:1. The advantage of a large ratio is a greater robustness and resistance towards mechanical breakage during storage; the advantage of a small ratio is an enhanced breakability in applications where a mechanical burst effect is desired.

The capsules according to the invention are preferably transparent and may resemble glassy beads which it is believed is aesthetically very different from conventional hydrogel capsules.

The capsules according to the invention may also be advantageously characterised by a Young's modulus of the shell material comprised between 0.5 GPa and 45 GPa, or preferably comprised between 5 GPa and 40 GPa.

The capsules according to the invention may also be advantageously characterised by having a mechanical rupture when compressed by a distance of 5 to 15 percent of its original size.

The present invention concerns also a method to confer, enhance, improve or modify the taste or odor properties of a flavoring or fragrance composition or of a flavored or perfumed article, which method comprises adding to said composition or article an effective amount of at least a capsule according to the invention. In the context of the present invention, "use of a capsule according to the invention" includes the use of any composition containing compound (I) and which can be advantageously employed in the flavor or perfume industry as active ingredient.

In another aspect, the invention provides a flavor or perfume composition comprising:
i) as perfuming or flavoring ingredient, at least one capsule according to the invention;
ii) at least one ingredient selected from the group consisting of a perfumery carrier and a perfumery base, or at least one ingredient selected from the group consisting of a flavor carrier and a flavor base; and
iii) optionally at least one perfumery or flavor adjuvant.

By "perfumery or flavor carrier" we mean here a material which is practically neutral from a perfumery or flavor point of view, i.e. that does not significantly alter the organoleptic properties of perfuming or flavoring ingredients. Said carrier may be a liquid or a solid.

As liquid carrier one may cite, as non-limiting examples, an emulsifying system, i.e. a solvent and a surfactant system, or a solvent commonly used in perfumery or flavors. A detailed description of the nature and type of solvents commonly used in perfumery or flavor cannot be exhaustive. However, one can cite as non-limiting example solvents such as dipropyleneglycol, diethyl phthalate, isopropyl myristate, benzyl benzoate, 2-(2-ethoxyethoxy)-1-ethanol or ethyl citrate, which are the most commonly used. As non-limiting examples of solvents commonly used in flavors, one can cite compounds such as propylene glycol, triacetine, triethyl citrate, benzylic alcohol, neobee, ethanol, sucrose esters such as sucrose oleate or sucrose erucate, vegetable oils or terpenes. Ethanol and sucrose esters are particularly preferred.

As solid carrier one may cite, as non-limiting examples, absorbing gums or polymers, or yet encapsulating materials. Examples of such materials may comprise wall-forming and plasticizing materials, such as mono, di- or trisaccharides, natural or modified starches, hydrocolloids, cellulose derivatives, polyvinyl acetates, polyvinylalcohols, proteins or pectins, or yet the materials cited in reference texts such as H. Scherz, Hydrokolloids : Stabilisatoren, Dickungs- und Gehermittel in Lebensmittel, Band 2 der Schriftenreihe Lebensmittelchemie, Lebensmittelqualitat, Behr's VerlagGmbH & Co., Hamburg, 1996. The encapsulation is a well known process to a person skilled in the art, and may be performed, for instance, using techniques such as spray-drying, agglomeration or yet extrusion ; or consists of a coating encapsulation, including coacervation and complex coacervation techniques.

By "perfumery or flavor base" we mean here a composition comprising at least one perfuming or flavoring co-ingredient.

Said perfuming or flavoring co-ingredient is not of the formula (I). Moreover, by "perfuming or flavoring co-ingredient" it is meant here a compound, which is used in perfuming or flavoring preparation or composition to impart a hedonic effect. In other words such a co-ingredient, to be considered as being a perfuming or flavoring one, must be recognized by a person skilled in the art as being able to impart or modify in a positive or pleasant way the odor or taste of a composition, and not just as having an odor or taste.

The nature and type of the perfuming or flavoring co-ingredients present in the base do not warrant a more detailed description here, which in any case would not be exhaustive, the skilled person being able to select them on the basis of its general knowledge and according to intended use or application and the desired organoleptic effect. In general terms, these perfuming or flavoring co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery or flavor. It is also understood that said co-ingredients may also be compounds known to release in a controlled manner various types of perfuming or flavoring compounds.

For the compositions which comprise both a perfumery carrier and a perfumery base, other suitable perfumery carrier, than those previously specified, can be also ethanol, water/ethanol mixtures, limonene or other terpenes, isoparaffins such as those known under the trademark Isopar® (origin: Exxon Chemical) or glycol ethers and glycol ether esters such as those known under the trademark Dowanol® (origin: Dow Chemical Company).

By "perfumery or flavor adjuvant" we mean here an ingredient capable of imparting additional added benefit such as a color, a particular light resistance, chemical stability, etc. A detailed description of the nature and type of adjuvant commonly used in perfuming or flavoring bases cannot be exhaustive, but it has to be mentioned that said ingredients are well known to a person skilled in the art.

An invention's composition consisting of at least one compound of formula (I) and at least one perfumery or flavor carrier represents a particular embodiment of the invention as well as a perfuming or flavoring composition comprising at least one compound of formula (I), at least one perfumery or flavor carrier, at least one perfumery or flavor base, and optionally at least one perfumery or flavor adjuvant.

Moreover, a capsule according to the invention can be advantageously incorporated into flavored articles to positively impart, or modify, the taste of said articles. Thus, in yet another aspect, the present invention provides a flavored article comprising:
i) as flavor ingredient, at least one capsule according to the invention, as defined above, optionally present as part of a flavoring composition; and
ii) a foodstuff base.

It is understood that in this case the active of the capsule is a flavor composition.

Suitable foodstuff bases, where the capsules of the invention are useful are typically those where a burst effect is desired. For instance, confectionary, cosmetics, breakfast cereal, oral care products such as toothpastes, baked goods, skin creams and savoury applications may be suitable media or end-products in which the capsules find utility.

In the context of the present invention "foodstuff' denotes a product that is taken orally and may either be swallowed. Therefore, a flavored article according to the invention comprises one or more compounds according to formula (I), as well as optional benefit agents, corresponding to taste and flavor profile of the desired edible product, e.g. a chocolate or a toothpaste.

The nature and type of the constituents of the foodstuffs or beverages do not warrant a more detailed description here, the skilled person being able to select them on the basis of his general knowledge and according to the nature of said product.

The proportions in which the compounds according to the invention can be incorporated into the various aforementioned articles or compositions vary within a wide range of values. These values are dependent on the nature of the article to be flavored and on the desired organoleptic effect as well as the nature of the co-ingredients in a given base when the capsules according to the invention are mixed with flavoring co-ingredients, solvents or additives commonly used in the art.

Furthermore, the invention's compound can also be advantageously used in all the fields of modern perfumery to positively impart or modify the odor of a consumer product into which said compound (I) is added. Consequently, a perfumed article comprising:
i) as perfuming ingredient, at least one capsule, as defined above; and
ii) a non-palatable consumer product base ;
is also an object of the present invention. It is understood that in this case the active of the capsule is a perfuming composition.

For the sake of clarity, it has to be mentioned that, by "non-palatable consumer product base" we mean here a non-edible consumer product, i.e which is not intended to be introduced in the mouth, and which is compatible with perfuming ingredients. In other words, a perfumed article according to the invention comprises the functional formulation, as well as optionally additional benefit agents, corresponding to a consumer product, e.g. a detergent or an air freshener, and an olfactive effective amount of at least one invention's compound.

The nature and type of the constituents of the non-palatable consumer product do not warrant a more detailed description here, which in any case would not be exhaustive, the skilled person being able to select them on the basis of its general knowledge and according to the nature and the desired effect of said product.

Examples of suitable non-palatable consumer product bases include solid or liquid detergents and fabric softeners as well as all the other articles common in perfumery, namely perfumes, colognes or after-shave lotions, perfumed soaps, shower or bath salts, mousses, oils or gels, hygiene products or hair care products such as shampoos, body-care products, deodorants or antiperspirants, air fresheners and also cosmetic preparations. As detergents there are intended applications such as detergent compositions or cleaning products for washing up or for cleaning various surfaces, e.g. intended for textile, dish or hard-surface treatment, whether they are intended for domestic or industrial use. Other perfumed articles are fabric softeners, fabric refreshers, ironing waters, papers, wipes or bleaches.

### Examples

The invention will now be described with reference to the following examples. It is to be understood that the examples are illustrative of the invention and that the scope of the invention is not limited thereto.

Samples according to the invention are denoted by a number and comparative examples by a letter. All amounts are % by weight unless otherwise indicated.

### Example 1

### Preparation of a Core-Shell Composite Structure According to the Invention

Core-shell capsule scaffolds were prepared made by complex coacervation with a gelatin/gum arabic system crosslinked with glutaraldehyde or transglutaminase.

Warm water fish gelatine (200 Bloom, supplied by Weishardt) and gum Arabic (Efficacia®, from CNI) were used as the hydrocolloids. A stock solution of gelatine (solution A) was prepared by mixing 180g of warm deionised water and 20g of gelatine in a vessel until completely dissolved; the solution was then maintained at 50°C. A stock solution of gum Arabic (solution B) was prepared by mixing 180g of cold deionised water and 20g of gum Arabic in a vessel until completely dissolved; the solution was then warmed and kept at 50°C. 105.4g of solution A was mixed with 100g of solution B in a vessel under gentle agitation (the gelatine/gum Arabic ratio being about 1:1). The pH was adjusted to 4.6 with a 50% w/w aqueous lactic solution. 70.3g of an active ingredient, limonene, was slowly added to this mixture and homogenised with a stirrer at 150 RPM during 5 minutes to reach an average droplet size of between 200 and 400µm.

The system was then diluted by the addition of 354.1g of warm deionised water, bringing the total hydrocolloid concentration to 3.4%w/w. The mixture was finally cooled to 20°C at a rate of 0.5°C.min⁻¹. The stirring speed was slightly decreased, the pH adjusted to 4.5 and 4.22g of transglutaminase (ACTIVA® WM, ex Ajinomoto) was added. Cross-linking was allowed to proceed overnight at 20°C. This results in an aqueous suspension of crosslinked microcapsule scaffolds. The slurry of capsule scaffolds was strained through a 0.5mm mesh size sieve and left on the mesh for 5 minutes. The separated scaffolds were then immersed in excess TEOS in a transparent sample dish (50 mm diameter) and observed by microscopy to evaluate whether and how the shell formed and incorporated silica over time.

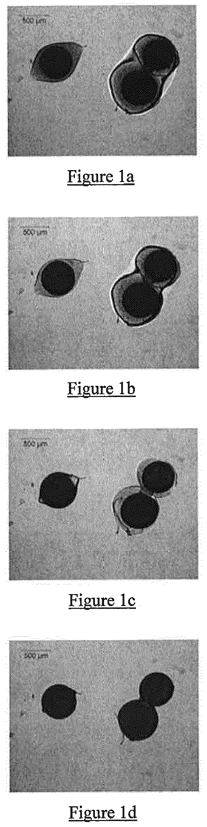

The results are shown in Figure 1 where Fig 1a shows formation at t=0, Fig 1b is at t=20 minutes, Fig 1c is at t=40 minutes and Fig 1d is at t=60 minutes). The results demonstrate that the thickness of the shell decreases over the course of 90 minutes, leaving behind a much denser hybrid shell composed of gelatin, gum arabic and silica.

Furthermore, the capsule shape obtained after 90 minutes remains unchanged even after 10 to 12 hours in the TEOS solution.

The final capsules retain their shape when suspended in water and, when stored in deionized water for ten days and then crushed between microscope slides, the capsules broke in a manner that can be described as 'brittle failure', resulting in 'glass-like' shell fragments. This is very different from the rupture associated with conventional coacervated particles.

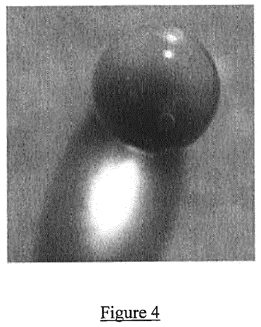

Once washed and dried on filter paper, the capsules appeared as large, nearly spherical beads with smooth and reflective surfaces (Fig. 4).

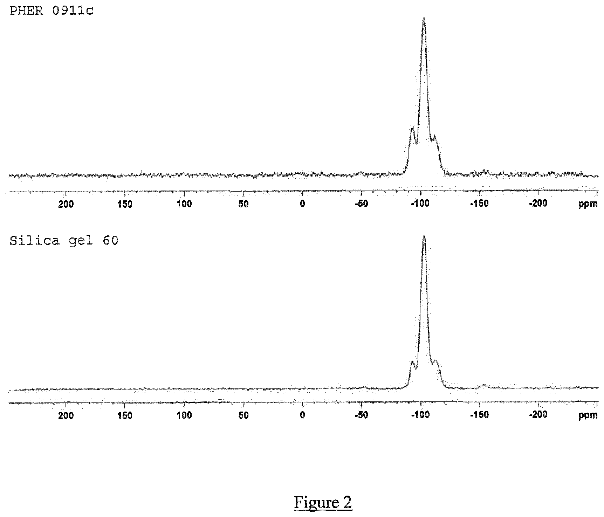

To confirm the presence of precipitated silica in the capsule shells, the shells were crushed and washed with isopropanol and water to ensure that no limonene was present (i.e. only the shell portion remained). Solid state NMR measurements were performed and compared with reference silica. The results, as shown in the following table and in Figure 2, confirmed that precipitation of silica in the hydrogel layer had occurred.

| | Composite shell* | Reference SiO₂** |
|---|---|---|
| Q2 (ppm) | -93.0 | -93.3 |
| Q3 (ppm) | -102.6 | -102.7 |
| Q4 (ppm) | -112.2 | -112.6 |

| | | |
|---|---|---|
| * (Figure 2, PHER 0911C) ** (Figure 2, silica gel 60) | | |

Standard-less energy dispersive x-ray spectroscopy (EDS) was performed using a scanning electron microscope (JSM-T330, JEOL Ltd. Tokyo, Japan) with a Noran Si(Li) detector and Vantage digital acquisition engine (Model 629J-1SPS, Thermo Electron Scientific Instruments LC, Madison, Wis., USA). Spectrum calibration was confirmed using an aluminium and copper standard (X-Checker EDS Performance Checker Mount, Structure Probe, Westchester, Pa. USA) prior to analysis. A cumulative spectrum was collected by sampling more than 10 fields. A low acceleration voltage (10 kV) was used in order to reduce the electron beam/specimen interaction volume.

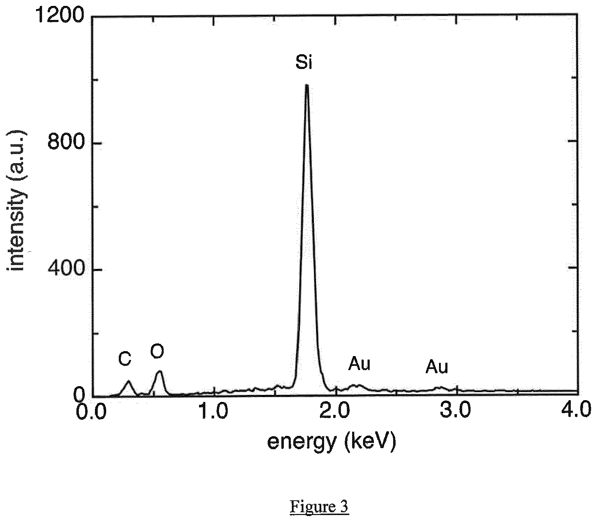

The EDS data, as shown in figure 3, demonstrates that the in-situ precipitation process yields a solid shell rich in silicon.

### Example 2

### Encapsulation of Mint Oil in a Core-Shell Capsule

The core-shell capsules were prepared in the manner described in Example 1 except that peppermint oil was used as the active ingredient.

### Example 3

### Encapsulation of Beta-Damascenone and of a Perfume Composition in a Core-Shell Capsule

The core-shell capsules were prepared in the manner described in Example 1 except that beta-damascenone was used as the active ingredient. Additionally, core-shell capsules were prepared in the same manner except that a perfume composition suitable for personal care applications was used as the active ingredient.

### Example 4

### Encapsulation of an Active Ingredient Embedded in a Solid Matrix in a Core-Shell Capsule

Core-shell capsules were prepared in the manner described in Example 1 except that a solid core material was used instead of liquid limonene. The core material was heated until molten and blended with an active ingredient (menthol) to form a molten mixture. The mixture was incorporated into the capsule in the manner described for the active ingredient in Example 1.

### Example 5

### Encapsulation of Mint Oil in a Core-Shell Capsule

Core-shell capsule scaffolds were produced by co-extruding a mint oil with sodium alginate into a CaCl₂ solution from a double capillary using standard methods known to the person skilled in the art. The resulting capsules were subsequently infused in an aqueous solution of 1 wt.-% lactic acid for 60 minutes; the capsules were then separated with filter paper and added to a liquid silica precursor (TEOS) and processed further as described in Example 1.

### Example 6

### Encapsulation of Orange Flavor in a Core-Shell Capsule and Application in a Breakfast Cereal

The core-shell capsules were prepared in the manner described in Example 1 except that a lemon flavor was used as the active ingredient. 5 g of dry capsules were then mixed into 50 g of an unflavored breakfast cereal (oatmeal flakes) and stored for two and sixteen weeks, respectively, under aerobic conditions in closed plastic cups at a temperature of 23°C.

For evaluation, product samples were prepared by adding 100 ml of cold milk to the dry blend 15 minutes prior to sensory testing.

A trained panel of 8 people were asked to evaluate the sample by first tasting only the milk brought into contact with the cereal, and then tasting the cereal itself. The participants were asked to answer the questions: 1. "Is there any flavor to be perceived in the milk alone?", 2. "Is there a flavor burst to be noticed upon chewing, and if yes would you describe the burst as 'strong' or 'weak'?". 8 out of 8 participants answered "No" to question 1 and "Yes, strong" to question 2.

### Example 7

### Encapsulation of Peppermint Oil in a Core-Shell Capsule; Use and Sensory Evaluation in a Chocolate Matrix

200 grams of black chocolate were melted in a water bath kept at 50°C, following which 10 grams of dry capsules, as prepared in Example 2, were blended into the molten chocolate with a spoon. An emulsifier (soy lecithin) was added to the slurry. The molten chocolate mass with the dispersed capsules was then poured into molds and kept in a refrigerator at 5°C during 24h and then stored at 23°C for 1 day.

For sensory evaluation, a trained panel of 8 people were asked to taste a sample of the chocolate containing peppermint-flavor capsules; they were informed beforehand that a flavor was added to the chocolate.

The participants were asked to first taste without chewing to assess whether any flavor has been released from the capsule into the chocolate mass. The participants were then asked to chew on the chocolate and assess whether a flavor burst is noticeable.

All participants were then asked the questions 1. "Did you perceive a non-chocolate flavor before chewing?"; and 2. "Did you perceive a non-chocolate flavor upon chewing?", with possible answers "Yes", "No", and "Not sure". Question 1 was answered as "No" by 6 out 8 participants. Question 2 was answered with "Yes" by 8 out of 8 participants. This result clearly shows that the capsules protect the peppermint flavor during the heating and mixing, and that a strong flavor burst effect is observed upon consumption.

The experiment was repeated except that the chocolate was stored at 23°C for 4 months. The same panellists were then asked the same questions with the result that question 1 was answered with "No" by 6 participants and with "Not sure" by 2 participants. Question 2 was answered with "Yes" by 8 out of 8 participants.

This result clearly demonstrates that the effect is retained after several months of storage.

### Example 8

### Encapsulation of Peppermint Oil in a Core-Shell Capsule; Use and Sensory Evaluation on a Confectionery Product

The core-shell capsules were prepared in the manner described in Example 1 except that a lemon flavor was used as the active ingredient. Cookies with chocolate glazing were purchased in a supermarket and kept at 40°C in an oven during 10 minutes to melt the glazing. Dry capsules were then sprinkled onto the molten glazing and the samples were left to cool in a refrigerator for 24 hours. A strong flavor burst was observed upon evaluation of the samples by a trained panel of 7 people.

### Example 9

### Encapsulation of Peppermint Oil in a Core-Shell Capsule; Use and Sensory Evaluation in a Toothpaste Base

Dry capsules, as prepared in Example 2, were mixed into two different toothpaste bases (gel and regular) and stored for 1, 4 and 16 weeks in small dishes suitable for observation with light microscopy. It was found that all capsules retained their structure during storage and released their content upon breakage, independent of storage time in the toothpaste base.

### Example 10

### Encapsulation of Peppermint Oil in a Core-Shell Capsule; Use and Sensory Evaluation in a Liquid Hand Soap Base

Dry capsules, as prepared in Example 2, were mixed into an unfragranced liquid hand soap base and stored for 1, 4 and 16 weeks and evaluated by light microscopy. It was found that all capsules retained their structure during storage and released their content upon breakage, independent of storage time in the hand soap base.

### Example 11

### Encapsulation of Peppermint Oil in a Core-Shell Capsule; Use and Sensory Evaluation in a Shower Gel Base

Dry capsules, as prepared in Example 2, were mixed into a shower gel base and stored for 1, 4 and 16 weeks and evaluated by light microscopy thereafter. It was found that all capsules retained their structure during storage and released their content upon breakage, independent of storage time in the shower gel base.

### Example 12

### Encapsulation of a Fragrance in a Core-Shell Capsule; Application in a Skin Cream Base

Dry capsules, as prepared in Example 3, were mixed into an unfragranced skin cream base and stored for 1, 4 and 16 weeks and evaluated by light microscopy thereafter. It was found that all capsules retained their structure during storage and released their content upon breakage, independent of storage time in the skin cream base.

### Example 13

### Encapsulation of Peppermint Oil in a Core-Shell Capsule; Comparison with Silica-only Core-Shell Capsule Made by a Traditional Sol-Gel Method

Core-shell capsules (Sample 13A) were prepared in the manner described in Example 2. In parallel, the same peppermint oil was encapsulated following a traditional method (as described for example in US2010/0143422A1) to form capsules with a silica-only shell (Sample 13B) as follows: 90g peppermint oil and 10 g TEOS were mixed and emulsified into 100g of 5 wt.-% solution of a surfactant (Tween 20, obtained from Sigma Aldrich) in deionized water, which has been acidified to pH 2 using a 20 wt.-% aqueous solution of lactic acid. The emulsification step was performed with a standard bench top rotor/stator homogenizer. The resulting emulsions were left to cure. Samples were withdrawn first after one day, and later at several days and weeks time (however, the results remained unchanged if the curing time was longer than a day).

Aliquots of both Sample 13A and Sample 13B were left to dry on filter paper overnight at room temperature. The amounts of peppermint oil retained in the capsules upon drying were then compared by thermogravimetric analysis, and it was found that the capsules of Sample 13A (Core-shell capsules made according to the present invention) contained 85 wt.-% volatile oil whereas Sample 13B (the silica microcapsules made according to the prior art) only contained less than 2 wt.-% volatile oil. The same samples were again analyzed after two months of storage at room temperature in an open container kept in a fume hood, and it was found that Sample 13A still contained 83 wt.-% volatile oil whereas all the volatile oil in Sample 13B was lost.

### Example 14

### Encapsulation of Peppermint Oil in a Core-Shell Capsule; Comparison with Silica-only Core-Shell Capsule Made by a Traditional Sol-Gel Method and with a Traditional Structure

Core-shell capsules (Sample 14A) were prepared in the manner described in Example 13. In parallel, the same peppermint oil was encapsulated according to a traditional method from the prior art (as described for example in US2010/0143422A1) to form silica-only capsules (Sample 14B) as follows: 25g TEOS were mixed with 8.5g deionized water and 22g ethanol; the pH of the mixture was adjusted to pH 2.1 with 15% HCl, mixed for 45 min to induce hydrolysis of the TEOS. A quantity of 12g of this mixture was then added to 28g peppermint oil, and this mixture was in turn emulsified in a 5 wt.-% solution of a surfactant as in Example 13, maintained at pH 2.1. The resulting emulsions were left to cure. Samples were withdrawn first after one day, and later at several days and weeks time, with identical results.

As in Example 13, the amounts of peppermint oil retained in the capsules upon drying were then compared by thermogravimetric analysis after one day and after two months, and it was found that the capsules of Sample 14A (Core-shell capsules made according to the present invention) contained 85 wt.-% volatile oil whereas Sample 14B (the silica microcapsules made according to the prior art) only contained 5.4 wt.-% volatile oil. After two months of storage at room temperature in an open container kept in a fume hood, and it was found that Sample 14A still contained 83 wt.-% volatile oil whereas all the volatile oil in Sample 14B was lost.

### Example 15

### Load after 15 Months of Storage and Comparison with Traditional Sol-Gel Microcapsules

Core-shell capsules according to the invention were prepared in the manner described in Examples 2 and 14 and compared to traditional silica microcapsules as described in Example 14. The volatile oil content of the dry capsules was re-analyzed after 15 months by thermogravimetric analysis and it was found that the aged capsules still contained 80.5 wt.-% of volatile oil (in contrast, all the volatile oil in the silica microcapsules had already been lost after 2 months). Even after 15 months of storage the capsule prepared according to the present invention were still brittle and provided a "burst" type release of the volatile oil upon mechanical fracturing.

### Example 16

### Mechanical Properties of Core/Shell Capsules and Comparison with Mechanical Properties of a Traditional Coacervate Capsule

Core-shell capsules with composite polymer/silica shells according to the invention were prepared in the manner described in Example 1. For comparison, polymer-only core-shell capsules were prepared by complex coacervation of the same gelatin/gum arabic polymers and following the same process until the crosslinking step, but without any of the steps involving silica as in example one, thus providing a sample of traditional coacervate capsules for comparison capsules (as described in the background and prior art section, for example as disclosed in FR1165805, The National Cash Register Company). Both samples were dried on filter paper and subjected to mechanical compression testing. For the mechanical compression test, a Physica MCR 300 platform (Anton Paar, Ostfildern, Germany) was used in vertical compression mode using a plate/plate geometry; the bottom surface was the standard Peltier plate of the instrument, kept at a constant temperature of 23°C, and the top plate with a diameter of 10 millimeters was mounted to the force transducer. A single capsule was positioned in the centre of the bottom plate and the top-driven transducer head was positioned above the capsule. To measure the mechanical behavior, the top plate was driven down at a constant speed of 10 micrometers per second and the normal force on the top plate was measured continuously. The size of the capsules was determined from the force-distance data as the vertical distance at which the top plate first comes into contact with the capsule. For each capsule the experiment was left to complete until the capsule burst under the applied deformation, evident in a sudden jump of the force-distance curve.

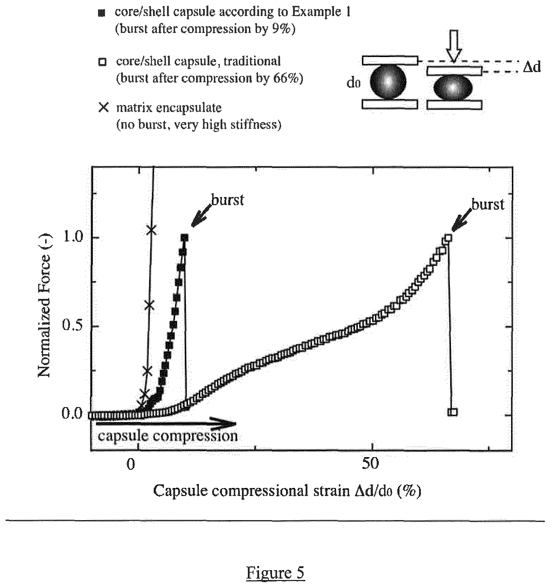

Examples for the resulting force-distance curves are shown in Figure 5 for a core/shell capsule made according the present invention (following Example 1) and a traditional coacervate capsule. (For Figure 5, the force data were normalized by the maximum force value, and the distance expressed as the capsule deformation ; the capsule deformation is the distance by which the capsule has already been compressed divided by the original size of the capsule, thus allowing comparison of different capsules in a well-defined manner).

Figure 5 demonstrates clearly that the core-shell capsules made according to the invention possess a high stiffness due to their composite silica/polymer shell (indicated by the steep slope of the force-deformation curve, which defines the stiffness), providing mechanical stability at low loads or deformations, yet they are able to burst and release their contents already at a compressive deformation as small as around 9%. Such mechanical behavior is highly desirable if a delivery system for flavors or fragrances needs to provide good barrier properties and stability during storage and processing, but is able to easily release its contents upon mechanical deformation.

In contrast, the traditional capsules are softer (indicated by the more shallow slope of the force-deformation curve), and they need to be compressed by more than half their original size to burst. Such behavior is not desirable in many applications, since the active ingredient could not be released easily, for example during mechanical deformation of the capsule during chewing or rubbing.

While the data shown in Figure 5 serve as an illustrative example, the same information was also analyzed quantitatively in more detail. For the samples studied, the force-deformation curves of fifty capsules each were compared. The results are summarized in Table 1. Clearly, the capsules made according to Example 1 in the present invention are much stiffer upon deformation, yet they allow easy release the active ingredient at a small much smaller deformation of the capsule. This property, which can be described as "brittleness", is highly desired for release of the active ingredient upon mechanical rupture.

Based on the data for capsule stiffness and size, the Young's modulus of the shell material ranges from 0.2 to 4.5 GPa for the traditional coacervate capsules and from 6.1 to 38 GPa for the core-shell capsules with silica/polymer shells made according to Example 1. The capsule made according to the invention therefore possess shells with a much higher Young's modulus; this means that even though they are easily broken under small applied deformation as desired, they are mechanically more resistant under small loads as compared to traditional coacervate capsules.

Surprisingly, these capsules still possess a very similar rupture force as compared to traditional capsules - this means that they remain very stable against small forces and even possess a greater stiffness below the rupture point as long as the capsules are not deliberately subjected to an external deformation, such as occurs during chewing or rubbing.

**Table 1 : Summary of mechanical properties of core-shell capsules according to the invention (all are mean values ± standard deviation, measured on 50 individual capsules)**

| **Property** | **Core-shell capsules with silica/polymer shells made according to Example 1** | **Traditional* core-shell capsules made by complex coacervation** |
|---|---|---|
| Stiffness (N/m) | 10817 ± 3621 | 3670 ± 651 |
| Rupture force (N) | 0.5939 ± 0.2699 | 0.5483 ± 0.1989 |
| Deformation at which capsule bursts (%) | 10.58 ± 5.57 | 46.78 14.73 |

| | | |
|---|---|---|
| *according to FR1165805 | | |

In summary, this example demonstrates that the core/shell capsules with silica/polymer shells according to the present invention provide desirable mechanical properties superior to those of traditional core-shell capsules made by complex coacervation.

### Example 17

### Comparison of Core-Shell Capsules according to the Invention with a Traditional Sol-Gel Microcapsule further encapsulated in a Polymer Matrix

The same core/shell capsules made according to the invention that were described in Example 1 and analyzed mechanically in Example 16 were also compared to a traditional matrix encapsulate of a sol-gel microcapsule in a polymer matrix made by spray-drying. The traditional sol-gel capsules were made exactly as sample 14B in Example 14, but instead of drying on filter paper, 10 grams of maltodextrin (DE 10) were added as matrix material to 50 grams of the sol-gel capsule suspension, and the mixture was spray-dried using a standard Büchi Laboratory spray-drier. This additional matrix may also be chosen among typical polymer suitable for matrix encapsulation by spray-drying of extrusion. Such polymers are well-known to the person skilled in the art and include, for example, but not limited to, maltodextrin, starches, modified starches, or gelatins. Force-deformation data are shown in Figure 5, along with the date described above in Example 16. The force-deformation of such matrix encapsulates containing sol-gel microcapsules are fundamentally different from the core/shell capsules made in this invention: the stiffness order of magnitude is higher (indicated by the very steep force-deformation curve), and it is not possible to bring the encapsulate to a burst, neither under small or large deformations. Additionally, force controlled experiments were performed and it was attempted to break the capsule by increasing the force further to a maximum of 7 N; in this case also, no burst occurred and the matrix encapsulate was compressed to tiny, flat tablet without having released the flavor. Therefore, whereas it was shown above in Examples 14 and 16 that traditional sol-gel capsules provide poor barrier properties and the active ingredient is lost, this example shows that further encapsulation of such traditional sol-gel capsules in a polymer matrix (as shown in D2) produces a delivery system completely unsuitable for release upon mechanical breakage and that is not a core/shell capsule.

## Claims

1. A process for preparing a core-shell capsule comprising the steps of:
(i) mixing a solid active ingredient and/or an oily liquid active ingredient with a polymeric material capable of forming a hydrogel shell around the active ingredient(s)
(ii) forming a shell comprising a hydrogel scaffold formed of a polymeric lattice around the core;
(iii) optionally cross-linking the polymeric lattice;
(iv) contacting the optionally cross-linked core-shell hydrogel shell with a liquid silica precursor so as to cause precipitation of silica within the scaffold structure thereby forming a composite shell of silica interspersed between the polymeric lattice.

2. A process according to claim 1 wherein the liquid silica precursor comprises dimethyldiethoxysilane, sodium silicates, hydrates of sodium silicates, TEOS or mixtures thereof.

3. A process according to claim 2 wherein the liquid silica precursor is TEOS and precipitation step (iv) is performed under acidic conditions.

4. A process according to any one of the preceding claims, wherein liquid silica precursor is in the form of an aqueous solution and the aqueous solvent phase present in the hydrogel scaffold is partially or completely consumed during hydrolysis and precipitation of the liquid silica precursor.

5. A process according to any one of the preceding claims, wherein a surfactant is used in the process

6. A process according to any one of claims 1 to 5, wherein a liquid silica precursor is mixed with the active ingredient before encapsulation.

7. A core-shell capsule wherein
(a) the shell is a composite comprising an organic and an inorganic phase, the organic phase comprising a network of polymeric strands and the inorganic phase comprising silica, and wherein the inorganic phase is physically interspersed between at least a proportion of the organic phase; and
(b) the core comprises a solid active ingredient and/or an oily liquid active ingredient;
and wherein said organic phase comprising a network of polymeric strands comprises a protein and a polymer.

8. A capsule according to claim 7, wherein the ratio of the core-shell capsule size to the thickness of the shell is preferably between 20:1 and 5:1.

9. A capsule according to any one of claims 7 to 8, wherein the thickness of the shell is from 10 to 50 micrometres.

10. A capsule according to any one of claims 7 to 9 wherein the active ingredient is an oily liquid having an interfacial tension against water of at least 0.001N/m.

11. A capsule according to any one of claims 7 to 10 wherein the Young's modulus of the shell material is comprised between 0.5 GPa and 45 GPa.

12. The use of silica physically interspersed in the shell portion of a hydrogel core-shell capsule comprising a liquid flavor or fragrance in the core portion to increase the rigidity and/or mechanical stability of the capsule.

13. A method to confer, enhance, improve or modify the taste or odor properties of a flavoring or fragrance composition or of a flavored or perfumed article, which method comprises adding to said composition or article an effective amount of at least a capsule according to any one of claims 7 to 11.

14. A flavor or perfume composition comprising:
i) as perfuming or flavoring ingredient, at least one capsule according to any one of claims 7 to 11;
ii) at least one ingredient selected from the group consisting of a perfumery carrier and a perfumery base, or at least one ingredient selected from the group consisting of a flavor carrier and a flavor base; and
iii) optionally at least one perfumery or flavor adjuvant.

15. A flavored article comprising:
i) as flavor ingredient, at least one capsule according to any one of claims 7 to 11; and
ii) a foodstuff base.

16. A perfumed article comprising:
i) as perfuming ingredient, at least one capsule according to any one of claims 7 to 11; and
ii) a non-palatable consumer product base ;
is also an object of the present invention.

## Patentansprüche

1. Verfahren zum Herstellen einer Kern/Hülle-Kapsel, umfassend die Schritte:
(i) Mischen eines festen Wirkstoffs und/oder eines öligen flüssigen Wirkstoffs mit einem polymeren Material, das eine Hydrogel-Hülle um den Wirkstoff (die Wirkstoffe) bilden kann;
(ii) Bilden einer Hülle, die ein Hydrogel-Gerüst umfasst, das um den Kern herum aus einem Polymergitter gebildet ist;
(iii) gegebenenfalls Vernetzen des polymeren Gitters;
(iv) Inkontaktbringen der gegebenenfalls vernetzten Kern/Hülle-Hydrogelhülle mit einem flüssigen Siliciumdioxid-Präkursor, um eine Ausfällung von Siliciumdioxid in der Gerüststruktur zu bewirken, wodurch eine Komposit-Hülle aus Siliciumdioxid gebildet wird, die zwischen dem polymeren Gitter eingestreut ist.

2. Verfahren nach Anspruch 1, wobei der flüssige Siliciumdioxid-Präkursor Dimethyldiethoxysilan, Natriumsilicate, Hydrate von Natriumsilicaten, TEOS oder Mischungen davon umfasst.

3. Verfahren nach Anspruch 2, wobei der flüssige Siliciumdioxid-Präkursor TEOS ist und der Fällungsschritt (iv) unter sauren Bedingungen ausgeführt wird.

4. Verfahren nach einem der vorhergehenden Ansprüche, wobei der flüssige Siliciumdioxid-Präkursor in Form einer wässrigen Lösung vorliegt und die im Hydrogel-Gerüst vorhandene wässrige Lösungsmittelphase während der Hydrolyse und Ausfällung des flüssigen Siliciumdioxid-Präkursors teilweise oder vollständig verbraucht wird.

5. Verfahren nach einem der vorhergehenden Ansprüche, wobei in dem Verfahren ein Tensid verwendet wird.

6. Verfahren nach einem der Ansprüche 1 bis 5, wobei ein flüssiger Siliciumdioxid-Präkursor vor dem Einkapseln mit dem Wirkstoff gemischt wird.

7. Kern/Hülle-Kapsel, wobei
(a) die Hülle ein Komposit ist, das eine organische und eine anorganische Phase umfasst, wobei die organische Phase ein Netzwerk von polymeren Strängen und die anorganische Phase Siliciumdioxid umfasst, und wobei die anorganische Phase physikalisch zwischen mindestens einem Teil der organischen Phase eingestreut ist; und
(b) der Kern einen festen Wirkstoff und/oder einen öligen flüssigen Wirkstoff umfasst;
und wobei die ein Netzwerk von polymeren Strängen umfassende organische Phase ein Protein und ein Polymer umfasst.

8. Kapsel nach Anspruch 7, wobei das Verhältnis der Größe der Kern/Hülle-Kapsel zur Dicke der Hülle vorzugsweise zwischen 20:1 und 5:1 liegt.

9. Kapsel nach einem der Ansprüche 7 bis 8, wobei die Dicke der Hülle 10 bis 50 Mikrometer beträgt.

10. Kapsel nach einem der Ansprüche 7 bis 9, wobei der Wirkstoff eine ölige Flüssigkeit mit einer Grenzflächenspannung gegen Wasser von mindestens 0,001 N/m ist.

11. Kapsel nach einem der Ansprüche 7 bis 10, wobei der Youngsche Modul des Materials der Hülle zwischen 0,5 GPa und 45 GPa liegt.

12. Verwendung von Siliciumdioxid, das physikalisch in den Hüllenteil einer Hydrogel-Kern/Hülle-Kapsel eingestreut ist, die ein flüssiges Aroma oder einen Duftstoff in dem Kernteil umfasst, um die Steifigkeit und/oder die mechanische Stabilität der Kapsel zu erhöhen.

13. Verfahren zum Vermitteln, Verstärken, Verbessern oder Modifizieren der Geschmacks- oder Geruchseigenschaften einer Aroma- oder Duftstoffzusammensetzung oder eines aromatisierten oder parfümierten Artikels, wobei das Verfahren die Zugabe einer wirksamen Menge mindestens einer Kapsel nach einem der Ansprüche 7 bis 11 zu einer solchen Zusammensetzung oder zu einem solchen Artikel umfasst.

14. Aroma- oder Parfümzusammensetzung, umfassend:
i) als parfümierenden oder aromatisierenden Bestandteil mindestens eine Kapsel nach einem der Ansprüche 7 bis 11;
ii) mindestens einen Bestandteil, der ausgewählt ist aus der Gruppe bestehend aus einem Parfümträger und einer Parfümeriebasis, oder mindestens einen Bestandteil, der ausgewählt ist aus der Gruppe bestehend aus einem Geschmacksträger und einer Geschmacksbasis; und
iii) gegebenenfalls mindestens einen Parfüm- oder Geschmacksstoff-Zusatzstoff.

15. Aromatisierter Artikel, umfassend:
i) als Aromabestandteil mindestens eine Kapsel nach einem der Ansprüche 7 bis 11; und
ii) eine Nahrungsmittelbasis.

16. Parfümierter Artikel, umfassend:
i) als parfümierenden Bestandteil mindestens eine Kapsel nach einem der Ansprüche 7 bis 11; und
ii) eine nicht schmackhafte Basis für Verbraucherprodukte;
ist ebenfalls ein Gegenstand der vorliegenden Erfindung.

## Revendications

1. Procédé de préparation d'une capsule à noyau-enveloppe comprenant les étapes consistant à:
(i) mélanger un ingrédient actif solide et/ou un ingrédient actif liquide huileux avec un matériau polymère capable de former une enveloppe d'hydrogel autour du ou des ingrédient(s) actif(s) ;
(ii) former une enveloppe comprenant une charpente d'hydrogel formée d'un maillage polymérique autour du noyau;
(iii) éventuellement, réticuler le maillage polymérique;
(iv) mettre en contact l'enveloppe d'hydrogel de la construction noyau-enveloppe éventuellement réticulée, avec un précurseur de silice liquide de façon à provoquer la précipitation de la silice au sein de la structure de charpente, formant ainsi une enveloppe composite de silice intercalée à travers le maillage polymérique.

2. Procédé selon la revendication 1, dans lequel le précurseur de silice liquide comprend du diméthyldiéthoxysilane, des silicates de sodium, des hydrates de silicates de sodium, du TEOS ou des mélanges de ceux-ci.

3. Procédé selon la revendication 2, dans lequel le précurseur de silice liquide est du TEOS et l'étape de précipitation (iv) est réalisée dans des conditions acides.

4. Procédé selon l'une quelconque des revendications précédentes, dans lequel le précurseur de silice liquide est sous la forme d'une solution aqueuse et la phase solvant aqueuse présente dans la charpente d'hydrogel est partiellement ou entièrement consommée pendant l'hydrolyse et la précipitation du précurseur de silice liquide.

5. Procédé selon l'une quelconque des revendications précédentes, dans lequel un tensioactif est utilisé dans le procédé.

6. Procédé selon l'une quelconque des revendications 1 à 5, dans lequel un précurseur de silice liquide est mélangé avec l'ingrédient actif avant encapsulation.

7. Capsule à noyau-enveloppe dans laquelle
(a) l'enveloppe est un composite comprenant une phase organique et une phase inorganique, la phase organique comprenant un réseau de brins polymères et la phase inorganique comprenant de la silice, et où la phase inorganique est physiquement intercalée à travers au moins une partie de la phase organique; et
(b) le noyau comprend un ingrédient actif solide et/ou un ingrédient actif liquide huileux;
et dans laquelle ladite phase organique comprenant un réseau de brins polymères comprend une protéine et un polymère.

8. Capsule selon la revendication 7, dans laquelle le rapport de la taille de la capsule à noyau-enveloppe à l'épaisseur de l'enveloppe est de préférence compris entre 20:1 et 5:1.

9. Capsule selon l'une quelconque des revendications 7 à 8, dans laquelle l'épaisseur de l'enveloppe est de 10 à 50 micromètres.

10. Capsule selon l'une quelconque des revendications 7 à 9, dans laquelle l'ingrédient actif est un liquide huileux ayant une tension interfaciale avec l'eau d'au moins 0,001 N/m.

11. Capsule selon l'une quelconque des revendications 7 à 10, dans laquelle le module d'Young du matériau d'enveloppe est compris entre 0,5 GPa et 45 GPa.

12. Utilisation de silice physiquement intercalée dans la partie enveloppe d'une capsule à noyau-enveloppe d'hydrogel comprenant un parfum ou arôme liquide dans la partie noyau pour augmenter la rigidité et/ou la stabilité mécanique de la capsule.

13. Procédé pour conférer, intensifier, améliorer ou modifier les propriétés de goût ou d'odeur d'une composition d'arôme ou de parfum ou d'un article aromatisé ou parfumé, lequel procédé comprend l'ajout à ladite composition ou audit article d'une quantité efficace d'au moins une capsule selon l'une quelconque des revendications 7 à 11.

14. Composition d'arôme ou de parfum comprenant:
i) en tant qu'ingrédient parfumant ou aromatisant, au moins une capsule selon l'une quelconque des revendications 7 à 11;
ii) au moins un ingrédient choisi dans le groupe constitué par un support de parfumerie et une base de parfumerie, ou au moins un ingrédient choisi dans le groupe constitué par un support d'arôme et une base d'arôme; et
iii) éventuellement, au moins un adjuvant d'usage courant en parfumerie ou dans l'industrie des arômes.

15. Article aromatisé comprenant:
i) en tant qu'ingrédient d'arôme, au moins une capsule selon l'une quelconque des revendications 7 à 11; et
ii) une base de produit alimentaire.

16. Article parfumé comprenant:
i) en tant qu'ingrédient parfumant, au moins une capsule selon l'une quelconque des revendications 7 à 11; et
ii) une base de produit de consommation non palatable;
qui est également un objet de la présente invention.
